Europäisches Patentamt

European Patent Office

Office européen des brevets

(19)

(11) **EP 0 958 785 B1**

(12) **FASCICULE DE BREVET EUROPEEN**

(45) Date de publication et mention
de la délivrance du brevet:
**31.03.2004 Bulletin 2004/14**

(51) Int Cl.⁷: **A61B 8/08**

(21) Numéro de dépôt: **99201465.4**

(22) Date de dépôt: **10.05.1999**

(54) **Procédé de détection de variations d'élasticité et appareil échographique pour mettre en oeuvre ce procédé**

Verfahren und Echographiegerät zur Erfassung von Elastizitätsveränderungen

Method and echographic apparatus for detecting variations in elasticity

(84) Etats contractants désignés:
**DE FR GB NL**

(30) Priorité: **19.05.1998 FR 9806311**

(43) Date de publication de la demande:
**24.11.1999 Bulletin 1999/47**

(73) Titulaire: **Koninklijke Philips Electronics N.V.**
**5621 BA Eindhoven (NL)**

(72) Inventeurs:
• **Cohen-Bacrie, Claude**
**75008 Paris (FR)**
• **Le Floch, Claire**
**75008 Paris (FR)**

(74) Mandataire: **Lottin, Claudine**
**Société Civile SPID,**
**156, Boulevard Haussmann**
**75008 Paris (FR)**

(56) Documents cités:
**US-A- 5 135 000**

• **KALLEL ET AL.: "Tissue elasticity reconstruction using linear perturbation method" IEEE TRANSACTIONS ON MEDICAL IMAGING, vol. 15, no. 3, juin 1996 (1996-06), pages 299-313, XP000587924 US**
• **YAMASHITA ET AL.: "Tissue elasticity reconstruction based on ultrasonic strain measurements" 1966 IEEE ULTRASONICS SYMPOSIUM PROCEEDINGS, vol. 2, novembre 1996 (1996-11), pages 1113-1117, XP000753532 US**

**Description**

L'invention concerne un procédé de détection de variations d'élasticité dans un tissu mou soumis à une compression externe de direction axiale prédéterminée.

L'invention concerne également un appareil échographique muni de moyens pour mettre en oeuvre ce procédé.

L'invention trouve son application dans l'industrie de l'imagerie médicale

Un procédé de reconstruction d'élasticité de tissu utilisant une méthode de perturbation linéaire est déjà connu de la publication intitulée « Tissue Elasticity Reconstruction Using Linear Perturbation Method » par F.Kallel et M. Bertrand, dans IEEE TRANSACTIONS ON MEDICAL IMAGING, VOL. 15, NO. 3, JUNE 1996, pp. 299-313. Cette publication décrit une méthode pour reconstruire le module élastique d'un tissu mou soumis à une compression externe statique à partir des mesures de déplacements engendrés par cette compression. Ce procédé met en oeuvre un algorithme connu de résolution d'un problème inverse, appelé algorithme de Newton-Raphson, qui utilise une relation directe donnant l'image d'un ensemble de champs de déplacements par un Modèle à Eléments Finis des équations d'élasticité et qui adapte cette relation directe, au sens des moindres carrés, pour fournir la répartition des modules d'élasticité correspondants. L'ensemble de champs de déplacements axiaux de tissus forme les données de départ qui sont préalablement estimées en utilisant une technique de corrélation en plusieurs bits appliquée à des signaux ultrasonores. Les problèmes relatifs à la matrice qui permet de résoudre le problème inverse selon l'algorithme de Newton-Raphson sont pris en compte en utilisant une technique connue de régularisation de Tikhonov utilisant la matrice identité I. L'utilisation d'une technique de régularisation est destinée à fournir un compromis entre la fidélité aux données observées et l'information A PRIORI de la solution. En utilisant un modèle de formation d'image échographique, cette publication enseigne que l'algorithme converge en 10 à 15 itérations. Des figures de la publication citée représentent des images de répartition de module élastique obtenues en 15 itérations par reconstruction à partir de données bruitées en utilisant l'algorithme de Newton-Raphson régularisé à chaque itération par le terme de Tikhonov avec I.

Dans le domaine médical de diagnostic d'anormalités dans les tissus mous, les tumeurs malignes se distinguent des tissus sains et des tumeurs bénignes par une différence d'élasticité de ces tissus, due à leur différence de structure. Dans le domaine du diagnostic du cancer du sein, il est particulièrement intéressant de disposer d'une méthode de mesure non invasive, et en même temps précise et fiable pour détecter des tumeurs malignes, aussi bien les tumeurs situées à fleur de peau que les tumeurs profondes, ainsi que des tumeurs débutantes de très petit diamètre qui sont très difficiles détecter.

Un problème est que la distribution du module d'élasticité dans un tissu mou n'est pas directement mesurable. Seul est mesurable le champ des déplacements dus à une compression du tissu mou. Mais ces déplacements sont extrêmement faibles d'où il résulte que leurs mesures ne sont pas directement exploitables par un praticien. Par contre la répartition des modules d'élasticité forme un ensemble de données tout à fait intéressantes du fait que ces données renseignent bien sur la nature des tissus et ont un contraste important facilement exploitable qui est lié aux mesures de déplacements dues à une compression du tissu mou. Un autre problème est que la répartition du module élastique n'est pas liée aux données du champ de déplacement par une relation directe. En revanche les données du champ de déplacement sont liées à la répartition du module élastique par une relation directe d'où il résulte que la répartition du module élastique dans un tissu doit être calculée par une méthode de résolution du problème inverse à partir des données du champ de déplacement. Une méthode de résolution d'un problème inverse est déjà décrite dans la publication citée. Un problème est que cette méthode connue est trop imprécise pour s'appliquer à la détection de tumeurs malignes par la détermination de la répartition du module élastique dans un tissu, du fait que les données mesurées du champ de déplacement sont très faibles et que la participation du bruit lors de l'acquisition de ces données est extrêmement forte.

C'est pourquoi la présente invention propose un procédé de détection selon la revendication 1.

Ce procédé fournit des images de reconstruction des variations d'élasticité dans le tissu qui sont beaucoup moins bruitées, beaucoup plus contrastées et qui permettent donc de détecter des très petites variations d'élasticité correspondant à des inhomogénéités du tissu et de très bien localiser ces défauts. Il en résulte une possibilité meilleure d'appliquer ce procédé à la détection des très petites tumeurs.

Ce procédé est mis en oeuvre avantageusement par un appareil échographique qui constitue un moyen non invasif d'aide au diagnostic.

L'invention est décrite ci-après en détail en référence aux figures schématiques annexées parmi lesquelles :

la FIG.1 représente par un diagramme de blocs les systèmes mettant en oeuvre les étapes du procédé de détection d'inhomogénéités dans un tissu ;
la FIG.2 représente par un diagramme de blocs les systèmes mettant en oeuvre les étapes fonctionnelles pour reconstruire la répartition du module d'élasticité à partir du champ de déplacement ;

la FIG.3 représente des lignes isodéplacement d'un champ de déplacements calculés à partir de signaux échographiques obtenus lors de la compression d'un tissu insonifié ;

la FIG.4 représente une image des variations du module élastique dans un tissu montrant deux inhomogénéités ;

les FIGs.5A, 5B représentent une image des variations du module d'élasticité obtenue par la méthode linéaire de Newton-Raphson régularisée selon une méthode de régularisation de l'invention dans le cas de rapports signal / bruit respectivement égaux à 50 et à 20, avec une seule itération et correspondant à la FIG.4 ;

les FIGs.6A, 6B représentent une image des variations du module d'élasticité obtenue par la méthode de Newton-Raphson régularisée selon la méthode de Tikhonov dans le cas de rapports signal / bruit respectivement égaux à 50 et 20 et correspondant à la FIG.4, pour comparer aux FIGs.5A, 5B.

L'invention concerne un procédé de traitement d'un signal échographique pour détecter des inhomogénéités dans un milieu insonifié c'est-à-dire soumis à des signaux d'ultrasons émis et reçus par un échographe. L'invention est fondée sur le traitement du signal échographique pour déterminer la répartition du module élastique dans le milieu insonifié en utilisant une méthode de résolution inverse régularisée, à partir de données d'un champ de déplacements mesurées dans ce milieu. L'invention trouve une application très intéressante dans la détection des tumeurs malignes par la détermination de la répartition du module élastique dans des tissus, ainsi que dans la détermination de l'étendue de ces tumeurs.

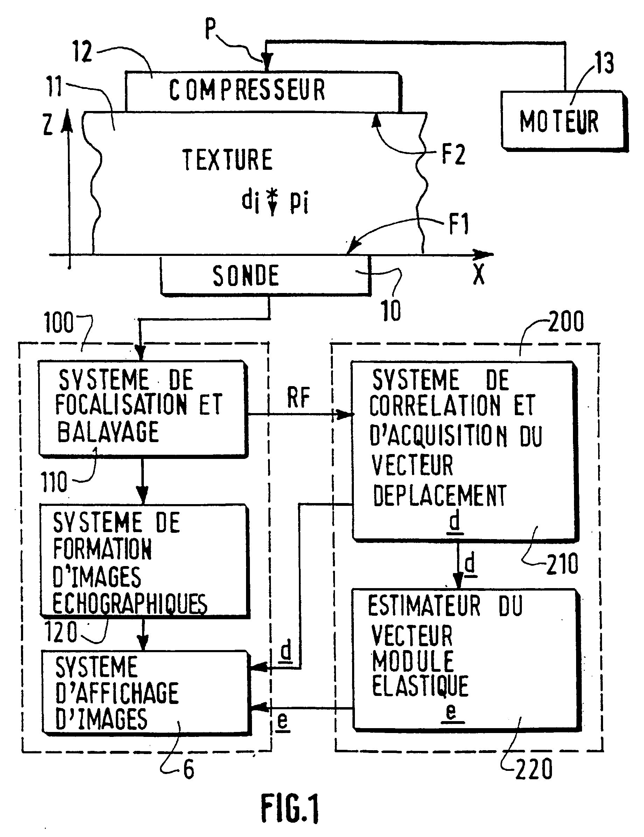

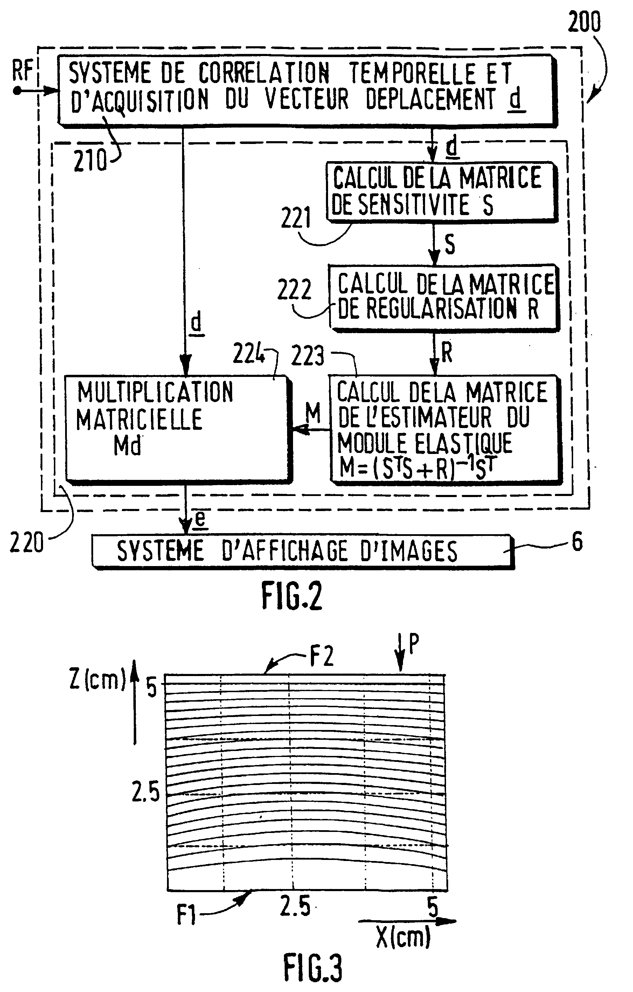

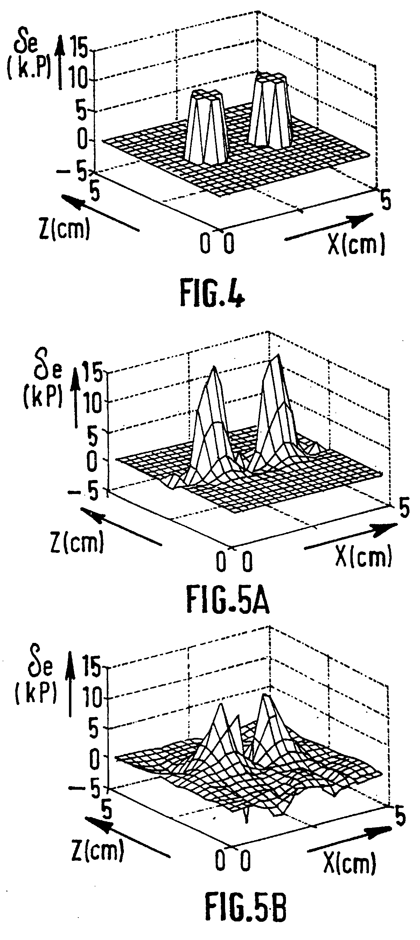

A titre d'exemple, en référence à la FIG.1, le milieu est un tissu mammaire référencé texture 11. Une pression P continûment variable est appliquée sur le tissu mammaire et une détermination d'un champ des déplacements est effectuée en soumettant une région du tissu à des tirs de signaux ultrasonores et en utilisant une méthode de corrélation appliquée aux signaux ultrasonores. D'une manière particulière, la pression P est continûment croissante et appliquée par le moyen d'une compresseur 12 actionné par un moteur 13. La zone 11 du tissu mammaire soumise à la compression présente une face fixe F1, sur laquelle est appliquée une sonde 10 d'un échographe 100, et une face parallèle F2 sur laquelle est appliquée perpendiculairement la pression P du compresseur. Dans le dispositif représenté sur la FIG.1, selon l'invention, la paroi mobile du compresseur 12 appliquée sur la face F2 se déplace à une vitesse donnée $\vec{v}$ en direction de la face F1. La sonde 10 appliquée sur la face fixe F1 envoie des signaux RF dans la texture 11 suivant des tirs parallèles en direction de la face F2. Les lignes de tirs selon l'axe Z sont perpendiculaires aux faces F1 et F2, et parallèles à la direction de pression du compresseur 12. Les tirs sont générés par l'échographe 100 qui comprend un système 110 de focalisation des signaux RF dans le tissu 11 parallèlement à un axe Z, et un balayage par les tirs de signaux RF linéairement parallèlement à un axe X parallèle à la face F1. Le balayage selon l'axe X est fait selon un nombre donné N de lignes de tirs avec une période de récurrence T. Lorsque les N tirs de 1 à N parallèlement à Z ont été effectués, la sonde recommence N tirs de 1 à N avec une période NT pour une ligne de tir donnée. La sonde reçoit en retour les signaux échographiques provenant de la texture 11 qui sont transmis à un système de détection de variation d'élasticité 200. De préférence, la sonde est une barrette de détecteurs linéaires émettant des tirs récurrents. Ce système 200 comprend un système 210 de corrélation temporelle en 1 bit pour effectuer en temps réel la corrélation de chacun des signaux transmis par la sonde provenant d'une localisation déterminée dans la texture 11. Le système de corrélation 210 effectue la corrélation des signaux relatifs à deux tirs successifs de la sonde effectués en une localisation donnée et fournit l'amplitude des déplacements de chaque point de la structure 11 insonifiée par la sonde sur chaque ligne de tir. Cette méthode fournit en continu et en temps réel durant l'action du compresseur 12 une image du champ de déplacement des structures formant la texture 11.

A cet effet l'image du champ de déplacements dans la zone de la texture 11 insonifiée est discrétisée. On ne stocke pas les signaux des différents balayages par la sonde. Ces signaux sont directement corrélés par le système de corrélation temporelle en 1 bit 210 qui fournit une image des déplacements de chaque point de la structure par exemple sous la forme de lignes isodéplacements, comme illustré par la FIG.3. Le présent procédé comprend en outre des moyens spécifiques pour rendre la détermination de la répartition du module élastique robuste au bruit, afin de bénéficier, en combinaison d'une reconstruction de la répartition du module élastique réalisée en temps réel, une telle reconstruction moins bruitée que selon l'état de la technique.

Il faut bien noter que les déplacements calculés par le système de corrélation 210 résultent des mesures directes des déplacements des structures de la région insonifiée au moyen de l'échographe. Ces déplacements sont radiaux, parallèles à la pression P et obtenus par l'action de cette pression P externe au tissu 11.

De manière préférentielle, plusieurs séries de balayages sont effectuées, avec entre chacune d'elles une compression égale ou un déplacement égal à $\vec{v}$ x NT de la face F2 du compresseur suivies d'un moyennage des données fournies par le système de corrélation 210. Cette méthode permet d'obtenir une grande précision dans cette étape d'acquisition 210 du champ de déplacements.

Les données du champ des déplacements sont calculées dans cette étape 210 sous forme de déplacements élémentaires $d_1$,... $d_i$,...$d_n$ relatifs à chaque pixel de l'image d'ultrasons discrétisée obtenue par la méthode de corrélation. Les données de déplacements élémentaires constituent un vecteur $\underline{d}$ de données de déplacements.

Ce vecteur déplacement $\underline{d}$ est lié à un vecteur appelé vecteur module élastique $\underline{e}$ (ou module d'Young) par une relation directe F qui s'écrit selon (1) :

$$\underline{d} = F(\underline{e}) \tag{1}$$

Le vecteur module élastique e est constitué de modules élastiques élémentaires $e_1,... e_i,....e_n$ correspondant respectivement aux déplacements élémentaires relatifs à chaque pixel $p_1,... p_i,....p_n$ de l'image discrétisée. Il est connu que $F(\underline{e})$ est l'image réalisée au moyen d'un Modèle à Eléments Finis de la répartition des modules élastiques élémentaires c'est-à-dire du vecteur $\underline{e}$. Un Modèle à Eléments Finis est une technique de calcul applicable aux équations d'élasticité et connue de l'homme du métier.

Le problème qui doit être effectivement résolu est la détermination du vecteur $\underline{e}$ donnant la répartition du module élastique dans la région insonifiée ayant fourni le champ de déplacement. La détermination du vecteur $\underline{e}$ permet de construire une image de répartition du module élastique formée de modules élémentaires relatifs à chaque pixel de l'image discrétisée.

La valeur du vecteur e est obtenu à partir de la relation (1) par la résolution du problème inverse selon la relation (2) :

$$\hat{e} = \arg\min[\|d - F(\underline{e})\|^2] \tag{2a}$$

où ê est un estimateur de $\underline{e}$. L'estimateur ê du module élastique $\underline{e}$ minimise la distance $\|d - F(\underline{e})\|^2$ entre les données $\underline{d}$ acquises par des mesures dans l'étape 210 de la FIG.1 et l'image $F(\underline{e})$ réalisée au moyen du Modèle à Eléments Finis. L'estimateur ê permet de déterminer le vecteur $\underline{e}$ qui, une fois injecté dans le Modèle à Eléments Finis donne une valeur de vecteur $\underline{d}$ aussi proche que possible du vecteur mesuré $\underline{d}$ dans l'étape 210.

La relation (2a) est connue de la publication citée au titre d'état de la technique et est référencée algorithme de Newton-Raphson. Cet algorithme permet de calculer le minimum de la distance $\|d - F(e)\|^2$ de manière itérative, par exemple, selon l'état de la technique avec 10 ou 15 itérations. L'algorithme de Newton-Raphson met en oeuvre une minimisation itérative de la distance exprimée par :

$$[d - F(\underline{e})]^T [\underline{d} - F(\underline{e})] \tag{2b}$$

Cette minimisation est effectuée par approximations linéaires au premier ordre successives de F. La première itération de cet algorithme permet de traiter le problème linéaire d'estimation du vecteur $\underline{e}$ en résolvant le problème inverse associé au problème direct dont l'équation est :

$$\underline{d} = S \underline{e} + b \tag{3}$$

où S est une matrice appelée matrice de sensibilité dont une méthode de calcul est décrite dans la publication citée au titre d'état de la technique et qui est une fonction de d, et où b est un bruit blanc gaussien. Il en résulte une nouvelle écriture de la première itération de l'algorithme de Newton-Raphson selon la relation suivante (4) :

$$\hat{e} = \arg\min [\underline{d} - S \underline{e}]^T [d - S \underline{e}] \tag{4}$$

Cet algorithme connu montre des inconvénients reconnus qui sont principalement des problèmes de convergence de l'algorithme et des problèmes d'instabilité de la solution liés au bruit qui est généralement important dans l'image de répartition des déplacements obtenue par ultrasons. Un problème inverse dont la solution est instable à cause du bruit s'appelle un problème mal posé. Selon la publication citée au titre de l'état de la technique, ce problème est résolu par la méthode de régularisation de Tikhonov qui consiste à appliquer un terme de régularisation à l'algorithme de Newton-Raphson (4) selon la relation (5) :

$$\hat{e} = \arg\min [(\underline{d} - S \underline{e})^T (\underline{d} - S\underline{e}) + \lambda \underline{e}^T I \underline{e}] \tag{5a}$$

Le terme de régularisation de Tikhonov qui s'écrit :

$$\lambda \; \underline{e} \; I^{T} \; \underline{e}$$

est une contrainte de «douceur» appliquée à l'algorithme de Newton-Raphson pour diminuer l'effet du bruit. Ce terme aplanit la solution de l'estimateur en affectant à tous les modules élastiques élémentaires $e_1, \ldots e_i, \ldots e_n$ des coefficients de régularisation tous égaux. Dit autrement, dans le terme de régularisation, la matrice de régularisation est la matrice identité I affectée de coefficients tous égaux. Cela signifie que, dans l'équation (3) où l'estimateur ê calcule un minimum, à la fois la distance et le terme de régularisation doivent être aussi faibles que possible. Le terme de régularisation de Tikhonov peut être écrit selon (7a) de manière connue de l'homme du métier :

$$\lambda \; \underline{e} \; I^{T} \; \underline{e} = \lambda [\, e_1^2 + e_2^2 + \ldots e_i^2 + \ldots + e_n^2 \,] = \lambda \sum_{i=1}^{i=n} e_i^2 \qquad (7a)$$

où les $e_i$ sont les modules élémentaires $e_1$ à $e_n$ du vecteur $\underline{e}$. L'algorithme de Newton-Raphson, auquel à chaque itération on applique la régularisation de Tikhonov sélectionne la solution où tous les $e_i$ sont poussés avec la même force vers zéro. Cette méthode de régularisation suppose que l'information contenue dans le champ de déplacement relative au module élastique d'un élément $e_i$ de $\underline{e}$ est indépendante de la localisation de $e_i$ concrétisée par l'indice i. On ne se trouve malheureusement jamais dans cette situation idéale.

C'est pourquoi selon l'invention, on propose une nouvelle méthode de régularisation très robuste au bruit pour appliquer à l'algorithme de Newton-Raphson. Parmi les problèmes que la présente invention cherche à résoudre, se trouvent le fait que la mesure du champ des déplacements liés au vecteur module élastique par la relation directe (1) est affectée de bruit et le fait que cette mesure dépend de la zone de la région insonifiée où ils ont été sont acquis : ainsi on ne peut pas traiter de façon indifférenciée les données provenant des différentes zones insonifiées. La méthode de régularisation de l'invention repose donc sur le constat qu'il existe dans la région insonifiée des zones qui contiennent des informations à un niveau plus élevé que d'autres zones. En conséquence la méthode de régularisation selon l'invention est basée sur une détermination de coefficients ou poids à affecter à chaque élément du vecteur module élastique pour tenir compte de la variabilité des mesures de déplacement et de la confiance que l'on accorde à ces informations dû à leur niveau résultant de ces mesures et dû à leur localisation dans la région insonifiée.

Dans la formule (5a) selon l'état de la technique, l'algorithme de Newton-Raphson régularisé par le terme de Tikhonov effectue à chaque itération la minimisation d'une fonction de coût C qui s'écrit :

$$C = [(\underline{d} - S\,\underline{e})^{T} (\underline{d} - S\underline{e}) + \lambda \; \underline{e}^{T} I \; \underline{e}] \qquad (6)$$

et qui permet d'écrire l'algorithme de Newton-Raphson régularisé par le terme de Tikhonov sous une nouvelle forme :

$$\hat{e}_i = \arg\min \, [S^{T}S + \lambda I]^{-1} \, S^{T}d \qquad (5b)$$

Le terme de régularisation de Tikhonov qui était

$$\lambda I \qquad (8a)$$

dans la formule $\hat{e}_i$ (5b) de l'algorithme connu de Newton-Raphson est transformé selon l'invention en une nouvelle matrice de régularisation notée

$$R \qquad (8b)$$

Selon l'invention, le terme de régularisation est formé en utilisant une matrice R différente de la matrice identité I multipliée par la constante $\lambda$ de Tikhonov et en calculant explicitement les coefficients notés $\alpha_i$ de cette nouvelle matrice de régularisation (R). Ceci est différent de la régularisation de Tikhonov où les coefficients de la matrice de régularisation I montrés par la relation (6) sont tous égaux. Ainsi, selon l'invention, les coefficients $\alpha_i$ de la matrice de régularisation R sont potentiellement tous différents pour être adaptés aux données $d_i$ du champ de déplacement mesurées en chaque pixel de la région insonifiée, en sorte que chaque point $e_i$ de l'image de reconstruction de la répartition du module élastique est formé d'un élément $\alpha_i\, e_i$ du vecteur module élastique qui est spécifiquement adapté à une donnée $d_i$ correspondante du champ de déplacement.

Il en résulte que l'algorithme de Newton-Raphson régularisé selon l'invention s'écrit selon la formule (9a) suivante :

$$\hat{e}_R = \arg\min\,[(\underline{d} - S\,\underline{e})^T\,(\underline{d} - S\,\underline{e}) + \underline{e}^T\,R\,\underline{e}] \tag{9a}$$

qui peut s'écrire selon (9b) :

$$\hat{e}_R = \arg\min\,[S^T S + R]^{-1}\,S^T d \tag{9b}$$

Selon l'invention, le terme de régularisation R peut ainsi être écrit selon la relation (7b) en utilisant les moyens d'écriture de la relation (7a) d'où il résulte que :

$$e^T R\underline{e} = \,_1e_1^2 + \,_2e_2^2 + \ldots \,_ie_i^2 \ldots + \,_ne_n^2 \approx \sum_{i=1}^{i=n} \,_ie_i^2 \tag{7b}$$

Cette relation (7b) exprime que les éléments diagonaux de la matrice R constituent les coefficients $\alpha_i$ appliqués respectivement sur chaque éléments $e_i$ du vecteur module élastique $\underline{e}$.

En référence à la FIG.2, l'invention propose un système électronique 200 associé à l'échographe, apte à travailler en temps réel, pour mettre en oeuvre une méthode avantageuse de détermination de ces coefficients $\alpha_i$ pour former des coefficients ou poids à appliquer aux éléments $e_i$. Ce système 200 met en oeuvre :

une étape 210 d'acquisition du champ de déplacement ou vecteur déplacement $\underline{d}$ par corrélation temporelle en 1 bit des signaux RF transmis par la sonde;
une étape 220 d'estimation du vecteur module élastique $\underline{e}$ qui comprend :
une opération 221 de calcul de la matrice de sensibilité S comme connu par exemple de la publication citée ;
une opération 222 de calcul de la matrice de régularisation R incluant :
une sous-opération de décomposition tronquée en valeurs singulières de la matrice de sensibilité S. On rappelle que l'opération de décomposition d'une matrice en valeurs singulières consiste à écrire la matrice en question dans une base de vecteurs propres comme connu de l'homme du métier. Une telle décomposition définit des nouvelles matrices dont une matrice de changement de repère notée V et une matrice diagonale notée $\Delta$ formée d'éléments notés $\delta_{ii}$. Selon l'invention, le niveau de troncature pour opérer la décomposition tronquée a été déterminé empiriquement et trouvé avantageusement de l'ordre de 0,2 à 10 %. La matrice diagonale $\Delta$ est utilisée pour construire une nouvelle matrice diagonale K dont les éléments diagonaux sont calculés par la formule (11a) suivante :

$$K_{ii} = (\Delta_{ii}/cte)\,) - \Delta_{ii}^2 \tag{11a}$$

une sous-opération de calcul proprement dit de la matrice de régularisation R selon l'invention qui est une matrice diagonale dont les éléments diagonaux $\alpha_{ii}$, aussi notés $\alpha_i$, sont les éléments diagonaux d'une matrice J non nécessairement diagonale obtenue par la formulation (11b) suivante :

$$J = V\,K\,V^T \tag{11b}$$

Selon l'invention, le terme de régularisation adoucit la répartition des éléments de module élastique au moyen d'un vecteur formé des coefficients diagonaux de la matrice R qui force certains pixels de l'image à avoir des éléments $e_i$ qui tendent plus fortement vers zéro que d'autres. La fonction de régularisation selon l'invention délimite un intervalle uniforme et force chaque valeur $e_i$ à rester dans cet intervalle centré sur une valeur moyenne $e_{Mi}$ spécifique de cet $e_i$. Ainsi, la définition de la matrice de régularisation R selon l'invention permet d'obtenir que le vecteur constitué des écarts à la moyenne des $e_i$, reste uniforme. Ce procédé de régularisation permet d'obtenir une image reconstruite de la répartition du module $\underline{e}$ de l'objet au moyen d'un estimateur ê de $\underline{e}$ dont la sensibilité au bruit est uniformément répartie sur chacune des composantes $e_i$ de $e_1$ à $e_n$ de $\underline{e}$. Les coefficients diagonaux de la matrice de régularisation R pondèrent respectivement les composantes élémentaires $e_i$ de manière potentiellement différente en fonction de leur localisation i. Ce procédé de régularisation est très robuste au bruit. Selon l'invention l'intervalle uniforme est défini et la matrice de régularisation R est dérivée de la matrice de sensibilité S par les calculs matriciels simples, décrits plus hauts, qui sont effectués dans le système 200 comme montré sur la FIG.1.

A l'issue de l'opération 222 de calcul de la matrice R, le système électronique 200 associé à l'échographe effectue :

une opération 223 de calcul de la matrice M de l'estimateur de $\underline{e}$ à partir de la relation (9b) selon la relation :

$$M = [S^T S + R]^{-1} S^T \qquad (10)$$

puis une opération 224 de multiplication matricielle de la matrice M par le vecteur $\underline{d}$.

Selon l'invention une seule itération suffit pour obtenir une image de reconstruction du module élastique $\underline{e}$ à partir du vecteur déplacement $\underline{d}$ par le procédé représenté par des blocs fonctionnels sur la FIG.1 et mis en oeuvre par le système électronique 200 associé à l'échographe 10, 100 de la FIG.1. Le terme de régularisation est donc appliqué à cette seule itération. D'où la simplicité de la mise en oeuvre de l'invention, comme illustré par la FIG.2 associée à la FIG.1.

Les FIGs.4, 5A, 5B et 6A, 6B illustrent les résultats obtenus par la méthode de l'invention. Ces FIGs peuvent être affichées au moyen d'un système d'affichage 6 comportant un moniteur, avec éventuellement des moyens d'enregistrement, associés à l'échographe 100. Ce système d'affichage est par exemple le même que celui qui permet l'affichage et l'enregistrement des images échographiques par l'intermédiaire du système de formation d'images 120.

La FIG.4 montre, dans un tissu une zone insonifiée discrétisée de largeur selon X de 5 cm et d'épaisseur selon Z de 5 cm. Ce tissu comprend deux inclusions ou inhomogénéités qui résultent en des variations du module élastique notées δe en kilopascals et qui apparaissent sous forme de deux reliefs réguliers.

Les FIGs.5A et 5B sont des reconstructions avec la matrice de régularisation R de l'invention à partir de données de déplacement déterminées selon la méthode échographique illustrée par les FIGs. 1 et 2 de variations e du module élastique, avec les mêmes unités, dans le cas où en outre les données de déplacement contiennent du bruit. La FIG.5A a été établie pour un rapport signal / bruit égal à 50 et la FIG.5B pour un rapport signal / bruit égal à 20.

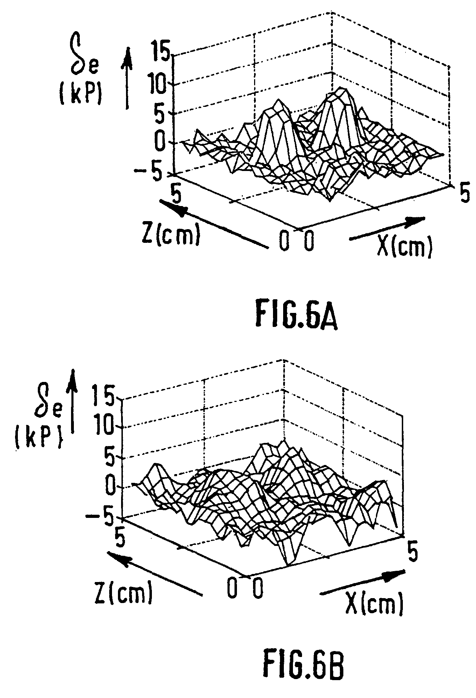

Les FIGs 6A et 6B sont des reconstructions avec le terme de régularisation de Tikhonov, réalisées dans les mêmes autres conditions que les FIGs.5A et 5B.

En comparant, dans les FIGs 5A et 6A (rapport signal / bruit égal à 50) les zones de tissu homogène entre les inhomogénéités, on peut constater que la méthode selon l'invention permet de complètement débruiter ces zones, alors que ces zones sont très bruitées par la méthode de régularisation de Tikhonov.

Le résultat est que les images reconstruites selon l'invention sont mieux contrastées et que les variations d'élasticité dues à des inclusions ou à des inhomogénéités du tissu sont mieux localisées et plus facilement détectées.

De même, dans les FIGs 5B et 6B (rapport signal / bruit égal à 20), en comparant les zones de tissus homogène entre les inhomogénéités, on peut constater que la méthode de l'invention offre encore un excellent contraste entre les différentes zones, alors que la méthode de régularisation de Tikhonov offre très peu de contraste, ce qui est un désavantage dans l'application à la détection de tumeurs.

Ainsi un appareil échographique muni des moyens de mise en oeuvre du procédé illustré par les FIGs 1 et 2, constitue un excellent moyen non invasif de détection de tumeurs, et particulièrement de tumeurs mammaires.

**Revendications**

1.   Procédé de détection de variations d'élasticité dans un tissu mou soumis à une compression externe de direction axiale prédéterminée, comprenant des étapes de :

estimation d'un champ de déplacements axiaux dans le tissu,
détermination d'un estimateur de module élastique incluant une opération de minimisation d'une distance entre une image d'une répartition de modules élastiques élémentaires par un Modèle à Eléments Finis et le champ de déplacements axiaux,

et régularisation de la solution de l'estimateur par une matrice diagonale (R) dont les coefficients $\alpha_{ii}$ sont des fonctions des déplacements axiaux ($d_i$) qui sont respectivement appliqués aux valeurs de modules élastiques élémentaires ($e_i$) pour forcer ces valeurs de modules élastiques élémentaires à rester dans un intervalle uniforme centré sur une valeur moyenne spécifique à chaque module élastique élémentaire.

2. Procédé selon la revendication 1, dans lequel l'estimateur de module élastique est calculé en utilisant une matrice (M) formulée par :

$$M = [S^T S + R]^{-1} S^T$$

où R est la matrice diagonale de régularisation et où S est une matrice de sensibilité qui est une fonction des déplacements axiaux, et en effectuant la multiplication matricielle de ladite matrice (M) par le vecteur de champ de déplacements ($\underline{d}$).

3. Procédé selon la revendication 2, où le tissu mou est couplé à une sonde ultrasonore associée à un échographe, cette sonde émettant et recevant des signaux échographiques parallèlement à la direction axiale de compression du tissu,

   et où l'estimation du vecteur champ des déplacements axiaux dans le tissu est effectuée en utilisant une technique de corrélation temporelle des signaux échographiques en 1 bit.

4. Appareil échographique pour détecter des variations d'élasticité dans un tissu mou incluant une sonde ultrasonore et des moyens de focalisation et balayage de la sonde qui est couplée à une surface de référence du tissu, et qui émet et reçoit des signaux échographiques parallèlement à une direction axiale du tissu, et des moyens de corrélation en 1bit des signaux échographiques, cet appareil comprenant :

   des moyens pour appliquer une compression continûment variable, externe au tissu, parallèlement à la direction radiale,
   des moyens pour estimer un champ de déplacements axiaux dans le tissu,
   un système estimateur de module élastique pour effectuer une opération de minimisation d'une distance entre une image de répartition de modules élastiques élémentaires par un Modèle à Eléments Finis et le champ de déplacement axiaux,
   et un système de régularisation de la solution de l'estimateur par une matrice diagonale (R) dont les coefficients ($\alpha_{ii}$) sont des fonctions des déplacements axiaux ($d_i$) et sont respectivement appliquées aux modules élastiques élémentaires ($e_i$).

5. Appareil selon la revendication 4, comprenant un système électronique pour calculer l'estimateur de module élastique en utilisant une matrice (M) formulée par :

$$M = [S^T S + R]^{-1} S^T$$

où R est la matrice diagonale de régularisation et où S est une matrice de sensibilité qui est une fonction des déplacements axiaux, et pour effectuer une multiplication matricielle de ladite matrice (M) par le vecteur de champ de déplacements ($\underline{d}$).

6. Appareil selon la revendication 5 comprenant un système électronique de traitement des signaux échographiques émis et reçus par la sonde pour effectuer une corrélation temporelle en 1 bit de ces signaux et pour fournir un champ des déplacements axiaux dans le tissu mou soumis à la compression externe axiale.

7. Appareil selon la revendication 6 dans lequel la sonde est une barrette de détecteurs linéaires émettant des tirs récurrents.

8. Appareil selon l'une des revendications 4 à 7 comprenant un système de formation d'images échographiques pour construire des images médicales à partir des signaux échographiques, un système d'affichage d'image pour afficher des images médicales du tissu et pour afficher des images de mesures de variations des déplacements du tissu soumis à la compression continûment variable et des images de reconstruction de la répartition du module élastique dans le tissu pour visualiser les variations d'élasticité dans ce tissu.

9. Appareil selon la revendication 8, pour l'aide au diagnostic de tumeurs dans les tissus, comprenant des moyens pour localiser les zones des images de reconstruction du module élastique présentant un substantiellement fort contraste des variations d'élasticité, pour détecter des tumeurs potentielles associées à ces zones.

10. Appareil selon la revendication 9, pour l'aide au diagnostic du cancer du sein, où le compresseur est muni de moyens pour compresser le sein en appliquant une pression radiale sur une face du tissu, avec une autre face du tissu parallèle servant de référence aux déplacements radiaux.

**Claims**

1. A method of detecting elasticity variations in a soft tissue which is subjected to an external compression in a predetermined axial direction, including the steps of:

estimating a field of axial displacements in the tissue,
determining an elasticity modulus estimator, including an operation for minimizing a distance between an image of a distribution of elementary elasticity moduli, by a Finite Element Model, and the field of axial displacements, and regularizating the solution of the estimator by means of a diagonal matrix (R) whose coefficients $\alpha_{ii}$ are functions of the axial displacements ($d_i$) which are applied to the respective values of the elementary elasticity moduli ($e_i$) in order to ensure that these elementary elasticity modulus values remain within a uniform interval which is centered around a mean value which is specific of each elementary elasticity modulus.

2. A method as claimed in Claim 1, in which the elasticity modulus estimator is calculated by utilizing a matrix (M) formulated as:

$$M = [S^T S + R]^{-1} S^T,$$

where R is the diagonal regularization matrix and where S is a sensitivity matrix which is a function of the axial displacements, and by performing the matrix multiplication of said matrix (M) by the displacement field vector ($\underline{d}$).

3. A method as claimed in Claim 2, in which the soft tissue is coupled to an ultrasonic probe associated with an echography apparatus, said probe emitting and receiving echographic signals parallel to the axial compression direction of the tissue, and in which the axial displacement field vector in the tissue is estimated while utilizing a 1-bit temporal correlation technique for the echographic signals.

4. An echography apparatus for detecting elasticity variations in a soft tissue, including an ultrasonic probe and means for focusing and scanning the probe which is coupled to a reference surface of the tissue and emits and receives echographic signals parallel to an axial direction of the tissue, and means for 1-bit correlation of the echographic signals, which apparatus includes:

means for applying a continuously variable, external compression to the tissue, parallel to the radial direction,
means for estimating a field of axial displacements in the tissue,
a system for estimating the elasticity modulus in order to perform an operation for minimizing a distance between an image of the distribution of elementary elasticity moduli, by a Finite Element Model, and the axial displacement field,
and a system for regularizing the solution of the estimator by means of a diagonal matrix (R) whose coefficients ($\alpha_{ii}$) are functions of the axial displacements ($d_i$) and are applied to respective elementary elasticity moduli ($e_i$).

5. An apparatus as claimed in Claim 4, including an electronic system for calculating the elasticity modulus estimator by utilizing a matrix (M) formulated as:

$$M = [S^T S + R]^{-1} S^T,$$

where R is the diagonal regularization matrix and where S is a sensitivity matrix which is a function of the axial displacements, and for performing a matrix multiplication of said matrix (M) by the displacement field vector (d).

6. An apparatus as claimed in Claim 5, including an electronic system for the processing of the echographic signals emitted and received by the probe in order to perform a 1-bit temporal correlation of these signals and to produce a field of axial displacements in the soft tissue subjected to the axial external compression.

7. An apparatus as claimed in Claim 6, in which the probe is an array of linear detectors emitting recurrent excitation signals.

8. An apparatus as claimed in one of the Claims 4 to 7, including a system for the formation of echographic images in order to compose medical images on the basis of echographic signals, an image display system for displaying medical images of the tissue and for displaying images of the measurements of variations of displacements of the tissue subjected to the continuously variable compression, and reconstruction images of the distribution of the elasticity modulus in the tissue in order to visualize the elasticity variations in the tissue.

9. An apparatus as claimed in Claim 8, intended to be used as a tool for the diagnosis of tumors in the tissues, comprising means for localizing the zones of the reconstruction images of the elasticity modulus presenting a substantially high contrast of the elasticity variations in order to detect potential tumors associated with these zones.

10. An apparatus as claimed in Claim 9, intended to be used as a tool for the diagnosis of breast cancer, where the compressor is provided with means for compressing the breast while applying a radial pressure to a surface of the tissue, another, parallel surface of the tissue serving as a reference for the radial displacements.

**Patentansprüche**

1. Verfahren zur Erfassung von Elastizitätsveränderungen in einem Weichgewebe, welches einem externen Druck vorbestimmter axialer Richtung ausgesetzt wird, beinhaltend die Schritte der:

Bewertung eines axialen Versetzungsfelds im Gewebe,
Bestimmung einer Bewertungsfunktion des Elastizitätsmoduls einschließlich einer Minimierungsoperation eines Abstands zwischen einem Aufteilungsbild von elementaren Elastizitätsmoduln durch ein Fertigelementemodell und dem axialen Versetzungsfeld, und
Regulierung der Lösung der Bewertungsfunktion durch eine Diagonalmatrix (R), deren Koeffizienten $a_{ii}$ axiale Versetzungsfunktionen ($d_1$) sind, die jeweils den Werten der elementaren Elastizitätsmoduln ($e_1$) zugeteilt werden, um diese Werte der elementaren Elastizitätsmoduln zu zwingen, in einem einheitlichen Intervall zentriert auf einem für jedes elementare Elastizitätsmodul spezifischen Mittelwert zu bleiben.

2. Verfahren nach Anspruch 1, in dem die Bewertungsfunktion des Elastizitätsmoduls unter Verwendung einer Matrix (M) berechnet wird, formuliert mit:

$$M = [S^T S + R]^{-1} S^T$$

wobei R die regulierende Diagonalmatrix und wobei S eine Sensibilitätsmatrix als Funktion axialer Versetzungen ist und wobei die Matrizenmultiplikation der besagten Matrix (M) über den Versetzungsfeldvektor (d) verläuft.

3. Verfahren nach Anspruch 2, wobei das Weichgewebe an eine Ultraschallsonde in Verbindung mit einem Echographen gekoppelt ist und diese Sonde Echographiesignale parallel zur axialen Richtung der Gewebekompression sendet und empfängt,
und wobei die Bewertung des axialen Versetzungsfeldvektors unter Verwendung einer zeitlichen Korrelation mit einem Bit durchgeführt wird.

4. Echographiegerät zur Erfassung von Elastizitätsveränderungen in einem Weichgewebe mit einer Ultraschallsonde und Mitteln zur Fokussierung und Abtastung der Sonde, die an eine Bezugsfläche des Gewebes gekoppelt ist und die echographische Signale parallel zu einer axialen Richtung des Stoffes sendet und empfängt, und Mitteln zur Korrelation mit einem Bit der Echographiesignale, wobei das Gerät enthält:

Mittel zum Anlegen einer kontinuierlich variablen Kompression außen am Gewebe und parallel zur radialen Richtung,

Mittel zur Bewertung eines axialen Versetzungsfelds im Gewebe,

ein Bewertungssystem des Elastizitätsmoduls zur Durchführung einer Minimierungsoperation eines Abstands zwischen einem Aufteilungsbild von elementaren Elastizitätsmoduln durch ein Fertigelementemodell und dem axialen Versetzungsfeld, und

ein Regulierungssystem der Lösung der Bewertungsfunktion durch eine Diagonalmatrix (R), deren Koeffizienten ($a_{ii}$) axiale Versetzungsfunktionen ($d_1$) sind, die jeweils den Werten der elementaren Elastizitätsmoduln ($e_1$) zugeteilt werden.

5. Gerät nach Anspruch 4 mit einem Elektroniksystem für die Berechnung der Bewertungsfunktion des Elastizitätsmoduls unter Verwendung einer Matrix (M), formuliert mit:

$$M = [S^T S + R]^{-1} S^T$$

wobei R die regulierende Diagonalmatrix und wobei S eine Sensibilitätsmatrix als Funktion axialer Versetzungen ist, und um eine Matrizenmultiplikation der besagten Matrix (M) über den Versetzungsfeldvektor ($\underline{d}$) durchzuführen.

6. Gerät nach Anspruch 5 mit einem Elektroniksystem für die Verarbeitung der Echographiesignale, die von der Sonde gesendet und empfangen werden, um eine zeitliche Korrelation dieser Signale mit einem Bit durchzuführen und um ein axiales Versetzungsfeld im Weichgewebe auszugeben, welches der axialen externen Kompression ausgesetzt ist.

7. Gerät nach Anspruch 6, in dem die Sonde eine Anordnung von linearen Detektoren ist, die rücklaufende Schallwellen aussenden

8. Gerät nach einem der Ansprüche 4 bis 7 mit einem echographischen Bildaufbausystem für den Aufbau medizinischer Bilder anhand von Echographiesignalen, ein Bildanzeigesystem zum Anzeigen der medizinischen Gewebebilder und zum Anzeigen der Messbilder der Versetzungsvariationen des dem ständig variablen Druck ausgesetzten Gewebes und zum Anzeigen der Rekonstruktionsbilder der Aufteilung des Elastizitätsmoduls im Gewebe zur Sichtbarmachung der Elastizitätsveränderungen in diesem Gewebe.

9. Gerät nach Anspruch 8 als Hilfe für die Diagnostik von Tumoren im Gewebe mit Mitteln zur Lokalisierung der Zonen derjenigen Rekonstruktionsbilder des Elastizitätsmoduls, die einen grundsätzlich starken Kontrast der Elastizitätsveränderungen aufweisen, um potenzielle mit diesen Zonen verbundene Tumoren zu erkennen.

10. Gerät nach Anspruch 9 als Hilfe für die Diagnostik von Brustkrebs, wobei der Kompressor mit Mitteln zum Komprimieren der Brust versehen ist, indem er einen radialen Druck auf eine Fläche des Gewebes ausübt, zu einer anderen Gewebefläche parallel, die den radialen Versetzungen als Bezugspunkt dient.

**FIG.1**

200

RF → SYSTEME DE CORRELATION TEMPORELLE ET D'ACQUISITION DU VECTEUR DEPLACEMENT $\underline{d}$

210

$\underline{d}$

CALCUL DE LA MATRICE DE SENSITIVITE S

221

S

CALCUL DE LA MATRICE DE REGULARISATION R

222

R

$\underline{d}$

224    223

MULTIPLICATION MATRICIELLE Md

M

CALCUL DE LA MATRICE DE L'ESTIMATEUR DU MODULE ELASTIQUE $M = (S^T S + R)^{-1} S^T$

220

$\underline{e}$

SYSTEME D'AFFICHAGE D'IMAGES    6

## FIG.2

Z(cm)    F2    P

5

2.5

F1    2.5    5

X(cm)

## FIG.3

FIG.4

FIG.5A

FIG.5B

**FIG.6A**

**FIG.6B**